(19) Europäisches Patentamt
European Patent Office
Office européen des brevets

(11) Veröffentlichungsnummer: **0 271 432 B1**

# (12) EUROPÄISCHE PATENTSCHRIFT

(45) Veröffentlichungstag der Patentschrift: **16.09.92**

(51) Int. Cl.$^5$: **C12P 41/00**, C12P 17/02, C12P 7/26, C12P 7/42, C12P 7/62, C07C 247/00

(21) Anmeldenummer: **87730144.0**

(22) Anmeldetag: **12.11.87**

(54) **Racematspaltung von 3-Acyloxy-Bicyclo[3.3.0]octan-7-on-2-carbonsäureestern durch stereospezifische enzymatische oder mikrobiologische Acylat-Hydrolyse.**

(30) Priorität: **13.11.86 DE 3638758**

(43) Veröffentlichungstag der Anmeldung: **15.06.88 Patentblatt 88/24**

(45) Bekanntmachung des Hinweises auf die Patenterteilung: **16.09.92 Patentblatt 92/38**

(84) Benannte Vertragsstaaten: **AT BE CH DE ES FR GB GR IT LI LU NL SE**

(56) Entgegenhaltungen:
**FR-A- 2 582 648**

**TETRAHEDRON, Band 42, Nr. 1, 1986, Seiten 435-444, Pergamon Press Ltd, GB; K. MORI et al.: "A new synthesis of ( + )-6a carbaprostaglandin I2 employing yeast reduction of a beta-keto ester derived from cis-bicyclo[3.3.0.]octane-3,7-dione as the key-step"**

**CHEM. PHARM. BULL., Band 33, Nr. 7, 1985, Seiten 2688-2696; K. KOJIMA et al.: "A convenient synthesis of ( + )-9(O)-methanoprostacyclin"**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Petzoldt, Karl, Dr.**
**Flachsweg 10**
**W-1000 Berlin 38(DE)**
Erfinder: **Dahl, Helmut, Dr.**
**Gollanczstrasse 102**
**W-1000 Berlin 28(DE)**
Erfinder: **Skuballa, Werner, Dr.**
**Olwenstrasse 25**
**W-1000 Berlin 28(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Verfahren zur stereospezifischen Acylat-Hydrolyse racemischer 3-Acyloxy-bicyclo[3.3.0]octan-7-on-2-carbonsäureester zu optisch aktiven 3-Alkoholen mit Hilfe von Enzymen oder Mikroorganismen.
Es eignet sich besonders zur Herstellung von optisch aktiven (+)-Bicyclo[3.3.0]octanol-derivaten der Formel (+)-I.

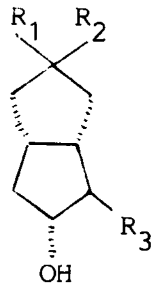

(+)-I,

worin
$R_1$ und $R_2$ gemeinsam ein Sauerstoffatom oder den zweibindigen Rest -O-X-O mit X als gerad- oder verzweigtkettigem Alkylen mit 1-7 C-Atomen oder $R_1$ und $R_2$ jeweils den Rest $OR_5$ mit $R_5$ als gerad- oder verzweigtkettigem Alkyl mit 1-7 C-Atomen und $R_3$ den Rest COOZ mit Z als Wasserstoffatom, gerad- oder verzweigtkettiges Alkyl mit 1-7 C-Atomen. Cycloalkyl mit 3-6 C-Atomen, Phenyl oder Aralkyl mit 7-10 C-Atomen oder
$R_3$ den Rest $-(CH_2)_n-O-COR_4$ mit n in der Bedeutung 1-4 und $R_4$ als gerad- oder verzweigtkettigem Alkyl mit 1 - 7 C-Atomen, Cycloalkyl mit 3-6 C-Atomen, Phenyl oder Aralkyl mit 7-10 C-Atomen, bedeuten.

Es ist dadurch gekennzeichnet, daß man racemische 3α-Acyloxy-cis-bicyclo[3.3.0]octan-derivate der Formel (±)-II

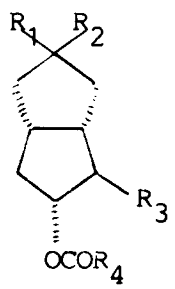

(±)-II,

worin $R_1$, $R_2$, $R_3$ und $R_4$ die oben angegebenen Bedeutungen haben, enzymatisch oder mikrobiologisch einer stereospezifischen Acylat-Hydrolyse unterwirft und das entstehende (+)-Bicyclo-[3.3.0]octanolderivat (+)-I vom unverseiften Bicyclo[3.3.0]octanol-acylat der Formel (-)-II abtrennt oder das unverseifte Enantiomere (+)-II vom verseiften Bicyclo[3.3.0]octanolderivat (-)-I abtrennt und anschließend einer chemischen Acylat-Hydro-lyse unterwirft.

Wenn X einen geradkettigen oder verzweigtkettigen Alkylenrest mit 1-7 C-Atomen bedeutet, so sind damit folgende Reste gemeint:
$-(CH_2)_n-$,mit n = 1-7 (Methylen, Ethylen, Tri-, Tetra-, Penta-, Hexa- und Heptamethylen), $-C(CH_3)_2-$, $-CH-(CH_3)-$, $-CH(CH_3)-CH_2-$, $C(CH_3)_2-CH_2-$, $-CH_2-CH(CH_3)-$, $CH_2-C(CH_3))_2$, $-CH_2-CH(CH_3)-)-CH_2-$, $-CH_2-C(CH_3)-_2-CH_2-$, $-CH-(C_2H_5)-$, $-C\ (C_2H_5)_2-$, $-CH(C_2H_5)-CH_2-$, $-C(C_2H_5)_2-CH_2-$, $-CH_2-CH-(C_2H_5)-$, $CH_2-C(C_2H_5)_2-$, $-CH_2-CH(C_2H_5)-CH_2-$, $-CH_2-C(C_2H_5)_2-CH_2-$ usw.
Z, $R_4$ und $R_5$ als geradkettige oder verzweigtkettige Alkylreste mit 1-7 C-Atomen bedeuten Methyl, Ethyl, n-Propyl, Isopropyl, n-Butyl, Isobutyl, sek.-Butyl, tert.-butyl, n-Pentyl, Isopentyl, sek.Pentyl, Neopentyl, n-Hexyl, Isohexyl, n-Heptyl, Isoheptyl,
Z bzw. $R_4$ als Aralkylreste mit 7-10 C-Atomen sollen folgende Reste umfassen: $-CH_2-C_6H_5$, $-CH_2-CH_2-$

$C_6H_5$, $-CH_2-CH_2-CH_2-C_6H_5$, $-CH_2-CH_2-CH_2-CH_2-C_6H_5$,

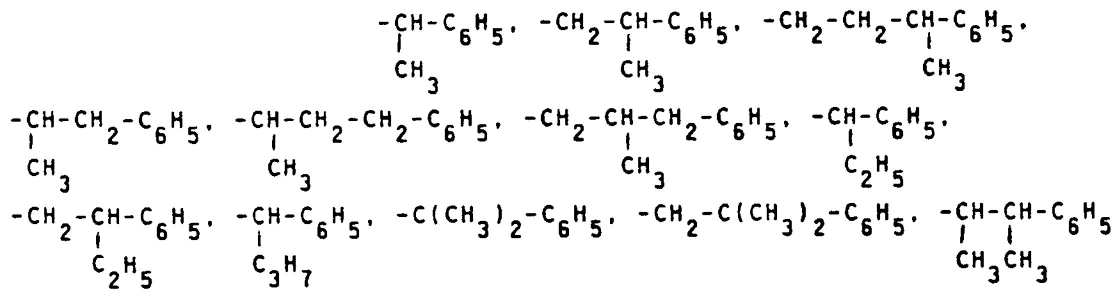

$-C(CH_3)_2-CH_2-C_6H_5$ usw.

Z bzw. $R_4$ als Cycloalkylreste mit 3-6 C-Atomen umfassen folgende Reste: Cyclopropyl, Cyclobutyl, Cyclopentyl, Cyclohexyl.

Optisch aktives 6a-Carba-prostacyclin und besonders einige davon abgeleitete Verbindungen besitzen also stabile Analoge des natürlichen Propstacylins ($PGI_2$) einen hohen therapeutischen Nutzen [R.C. Nickolson, M.H. Town, H. Vorbrüggen: Prostacyclin-Analogs Medicinal Research Reviews, Vol. 5, No. 1, S. 1-53 (1985)]. Die in dieser neueren Übersicht aufgeführten Synthesen sind lang und führen teilweise nur zu racemischen Carbacyclinen. Besonders aufwendig sind die Synthesen, die zu Carbacyclinen in der dem natürlichen $PGI_2$ entsprechenden absoluten Konfiguration führen. Das liegt daran, daß gut zugängliche, geeignete Ausgangsmaterialien achiral sind und die optische Aktivität erst im Syntheseverlauf in hierfür geeignete Zwischenstufen eingeführt werden muß.

Mehrere Synthesen gehen bereits von optisch aktiven 7α-Hydroxy-6B-hydroxymethyl-2-oxa-bicyclo-[3.3.0]octan-3-on-Derivaten aus. Damit ist zwar das Problem der Einführung der optischen Aktivität gelöst. Es müssen jedoch noch weitere vielstufige Synthesesequenzen für die Substitution der 2-Oxa-Funktion durch eine Methylengruppe durchgeführt werden, um zu Derivaten des 3α-Hydroxy-2ß-hydroxymethyl-bicyclo[3.3.0]-octan-7-ons zu gelangen, die sich erst für den Anbau der für die Carbacyclin-Analoga jeweils typischen α- und ω-Ketten eignen.

Eine neuere Publikation beschreibt die Verwendung von cis-Bicyclo[3.3.0]octan-3,7-dion-Derivaten zur Synthese optisch aktiver Carbacycline. Kojima et al. beschreiben in Chem. Pharm. Bull. 33, 2688 (1985) ein Verfahren, das die Trennung diastereomerer Salze der racemischen 7,7-Ethylendioxy-3α-hydroxy-cis-bicyclo[3.3.0]octan-2-carbonsäure einschließt.

Auch dieses Verfahren erfordert noch 7 Reaktionsschritte, um ausgehend von 3-Oxoglutarestern zum Startmaterial für Carbacyclin-Analoga zu gelangen. Zudem wird eine instabile ß-Ketosäure-Zwischenstufe durchlaufen.

Für die Herstellung optisch aktiver Carbacyclin-Analoga, wie sie oben beschrieben werden, ist weiterhin kein Syntheseweg, der eine einfache Herstellung gestattet, bekannt.

Als Enzyme eignen sich für das erfindungsgemäße Verfahren vorzugsweise
Lipase-PL aus Alcaligenes (Fa. Meito Sangyo)
Lipase My aus Candida cylindracea (Fa. Meito Sangyo)
Lipase "Saiken" aus Rhizopus (Fa. Nagase)
Lipase "Sclerotinia" (Fa. Nagase)
α-Chymotrypsin aus Rinderpankreas (Fa. Chemical Dynamics Corporation)
Alcalase T (Fa. Novo Industrias)
Esterase aus Schweineleber (Fa. Boehringer Mannheim)
Lipase aus Schweinepankreas (Fa. Chemical Dynamics Corporation)
Subtilisin aus Bacillus subtilis (Fa. Boehringer Mannheim),
wobei die Enzyme sowohl in gelöster, suspendierter oder z.B an BrCN-aktivierter Sepharose oder an Oxiranacrylperlen immobilisierter Form oder in irgendeiner anderen immobilisierten Form eingesetzt werden können.

Als Mikroorganismen eignen sich für das erfindungsgemäße Verfahren vorzugsweise
Alcaligenes marshallii ATCC 21030
Mucor rouxii ATCC 8097
Corynebacterium equi ATCC 21107
Trichoderma koningi CBS 85068
Sarcina lutea ATCC 9341

Penicillium citrinum ATCC 8506
Flavobacterium lutescens IFO 3085
Alcaligenes paradoxus ATCC 17713
Es können aber auch die aus den Mikroorganismen isolierten Enzyme in gelöster oder immobilisierter Form verwendet werden.

Die nach dem erfindungsgemäßen Verfahren herstellbaren optisch aktiven Bicyclooctan-Derivate der Formel (+)-I stellen wertvolle Zwischenprodukte für die Synthese pharmakologisch wirksamer Prostacyclinderivate dar. Die Mehrzahl der stereospezifisch hydrolysierenden Enzyme und Mikroorganismen verseifen die racemischen 3α-Acyloxy-cis-bicyclo[3.3.0]octan-derivate der Formel (±)-II zu optisch aktiven Alkoholen der Formel (+)-I, die in ihrer absoluten Konfiguration dem natürlichen Prostacyclin $PGI_2$ entsprechen. Dies ergibt sich durch Vergleich der spektralen und optischen Eigenschaften mit Vergleichssubstanzen, die aus Zwischenstufen einer Synthese optisch aktiver Carbacyclin-Analoga konventionell hergestellt werden können. Die so erhaltenen Produkte können nach Abtrennung des unverseiften "falschen" Enantiomeren (-)-I direkt zur Synthese von dem natürlichen Prostacyclin $PGI_2$ entsprechendenden Analoga eingesetzt werden.

Es gibt jedoch auch Mikroorganismen und Enzyme, wie z.B. α-Chymotrypsinoder Alcalase T, die von den beiden Komponenten des Racemats (±)-II das "falsche", unnatürlich konfigurierte Enantiomere zu (-)-I verseifen. In diesem Falle wird das zurückgebliebene unverseifte Enantiomere (+)-II für die Synthese $PGI_2$-analoger Carbacycline herangezogen.

Das erfindungsgemäße Verfahren arbeitet ansonsten nach allgemein bekannten Verfahrensbedingungen, die man üblicherweise bei enzymatischen Reaktionen bzw. mikrobiologischen Umwandlungen anwendet und die aus den Beispielen hervorgehen. Der Ablauf der enzymatischen oder mikrobiologischen Umwandlung wird durch Analyse laufend entnommener Proben verfolgt. Als Analysen-Methoden eignen sich HPLC oder dünnschichtchromatographische Schnellanalysen (Kieselgelplatten der +Fa. Merck/Darmstadt, Entwicklung mittels Ether, Anfärben mittels Schwefelsäure/Ethanol).

Die Reaktion wird abgebrochen und der Ansatz aufgearbeitet, wenn 50% des eingesetzten racemischen Substrates umgesetzt sind.

Die erfindungsgemäße enzymatische oder mikrobiologische stereo-spezifische Acylat-Verseifung eignet sich besonders für die Acylat-Verseifung folgender racemischer Prostacyclin-Zwischenstufen:

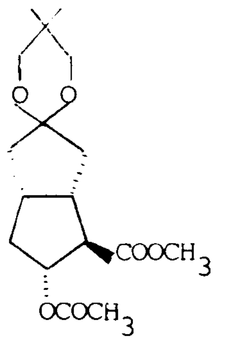

(+)-1

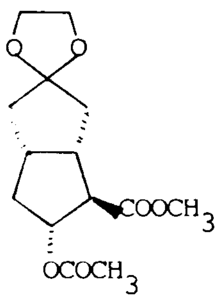

(+)-2

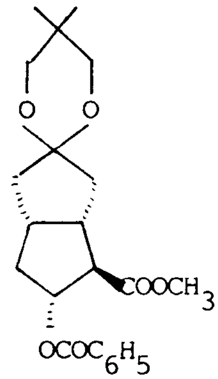

(+)-3

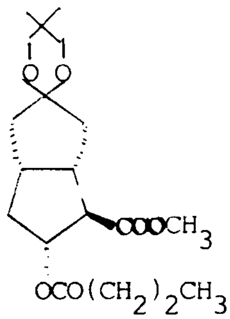

(+)-6

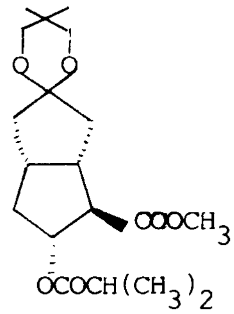

(+)-7

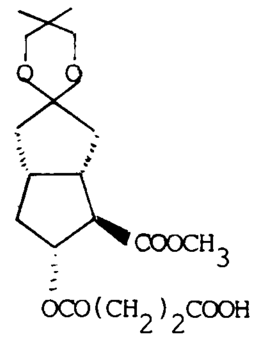

(+)-8

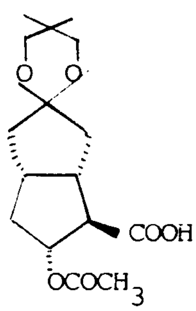

(+)-9

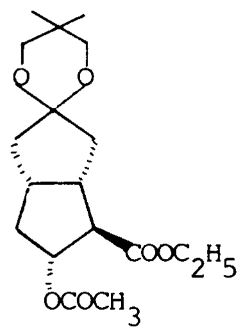

(+)-10

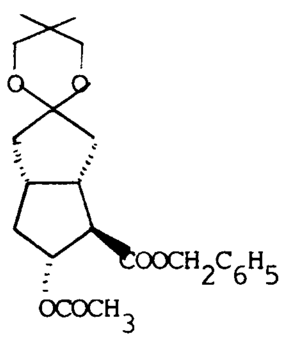

(+)-11

Aus den nach dem erfindungsgemäßen Verfahren hergestellten optisch aktiven 3α-Hydroxy-Verbindungen der allgemeinen Formel (+)-I lassen sich pharmakologisch wirksame Prostacycline herstellen. Beispielsweise gelangt man ausgehend von (+)-I zum Wirkstoff Iloprost (beschrieben in EP 11591).

Am 11.11.86 wurden die Stämme Mucor rouxii (DSM 3897) und Alcaligenes marshallii (DSM 3900) bei der Deutschen Sammlung von Mikroorganismen hinterlegt.

**Herstellung der Ausgangsverbindungen:**

Beispiel A 1

7,7-(2,2-Dimethyl-trimethylendioxy)-cis-bicyclo[3.3.0]octan-3-on-2-carbonsäuremethylester

Man suspendiert 38,34 g 55-60%iges Natriumhydrid in 616 ml Dimethylcarbonat, erwärmt unter Stickstoff auf 50 °C und gibt eine kleine Menge der Lösung von 49,31 g 3,3-(2,2-Dimethyltrimethylendioxy)-cis-bicyclo[3.3.0]octan-7-on in 370 ml Dimethylcarbonat dazu. Durch Zugabe von 2 ml Methanol induziert man die Reaktion, gibt die restliche Lösung dazu und rührt insgesamt 7,5 Std. bei 50 °C. Man kühlt im Eisbad, zersetzt überschüssiges Natriumhydrid mit Methanol, gibt Wasser hinzu und neutralisiert mit Essigsäure. Man extrahiert das Produkt mit Dichlormethan, konzentriert im Vakuum und kristallisiert das

Produkt mit Hexan. Man erhält 532,44 g Produkt vom Schmelzpunkt 72° C.

Beispiel A 2

7,7-(2,2-Dimethyl-trimethylendioxy)-3α-hydroxy-cis-bicyclo[3.3.0]octan-2ß-carbonsäuremethylester

**Methode A**

Man löst 52,0 g 7,7-(Diomethyl-trimethylendioxy)-cis-bicyclo[3.3.0]octan-3-on-2-carbonsäuremethylester in der Wärme in 1000 ml Methanol und kühlt auf -40° C ab. Dann trägt man 20,91 g Natriumborhydrid ein, rührt 30 Minuten, versetzt langsam mit 171 ml Aceton und neutralisiert nach einer weiteren Stunde mit Essigsäure. Nach Abdestillieren der Hauptmenge Lösungsmittel wird mit Wasser und Dichlormethan versetzt, die organische Phase mit Natriumsulfat getrocknet und im Vakuum konzentriert.

Den Rückstand nimmt man in 550 ml Methanol auf, fügt 9,94 g Natriummethylat dazu und erwärmt 105 Minuten auf 40° C. Man kühlt im Eisbad, neutralisiert und arbeitet wie vorher beschrieben auf. Das erhaltene Rohprodukt chromatographiert man an Kieselgel mit Dichlormethan-Ethylacetat-Gemischen. Man erhält 47 g der gewünschten Verbindung, die sich mit Hexan kristallisieren läßt und einen Schmelzpunkt von 43° C aufweist.

**Methode B**

56,6 g 7,7-(Dimethyl-trimethylendioxy)-cis-bicyclo[3.3.0]octan-3-on-2-carbonsäuremethylester werden in 300 ml Ethylacetat gelöst und nach Zugabe von 5,1 g Platindioxid bei 22° C unter Normaldruck hydriert, bis die Wasserstoffaufnahme beendet ist. Man filtriert vom Katalysator und konzentriert im Vakuum. Man erhält 56,8 g der gewünschten Verbindung, deren Reinheit zur Herstellung kristallisierender Ester ausreicht. Man kann das Produkt aus Hexan kristallisieren und erhält 44,4 g Erstkristallisat vom Schmelzpunkt 43° C.

Beispiele A 3

7,7-(2,2-Dimethyl-trimethylendioxy)-3α-hydroxy-cis-bicyclo[3.3.0]octan-2ß-carbonsäuremethylester-3-ester

a) Acetat (±)-1

Man löst 100 g des im vorigen Beispiel nach Methode B erhaltenen Rohprodukts in 57 ml Pyridin und 57 ml Essigsäureanhydrid und erwärmt 3 Stunden auf 40° C. Nach dem Abkühlen gibt man Eiswasser hinzu, extrahiert mit Dichlormethan, wäscht die organische Phase mit 2-normaler Schwefelsäure, Natriumhydrogen-carbonant-Lösung und Natriumchlorid-Lösung, trocknet mit Natriumsulfat, konzentriert im Vakuum und kristallisiert den Rückstand aus Hexan. Man erhält 97,6 g Produkt vom Schmelzpunkt 54° C.

b) Benzoat (±)-3

Man löst 5,0 g des Hydroxyester-Rohprodukts in 10 ml Pyridin, versetzt mit 2,47 ml Benzoylchlorid und rührt 4 Stunden bei 22° C. Dann gbit man langsam in 100 ml Eiswasser, rührt 30 Minuten und saugt das Kristallisat ab. Nach Trocknen und Umkristallisieren aus Methanol erhält man 5,97 g Produkt vom Schmelzpunkt 102° C.

c)Isobutyrat (±)-7

Man löst 4,265 g des kristallisierten Hydroxyesters in 40 ml Dichlormethan, versetzt mit 4,16 ml Triethylamin und langsam mit 3,14 ml Isobutyrylchlorid. Nach 2,5 Stunden bei 22° C werden 40 ml gesättigte Natriumhydrogencarbonat-Lösung zugegeben, die organische Phase getrennt und mit 2n-Schwefelsäure unter Eiskühlung und mit Wasser gewaschen, mit Natriumsulfat getrocknet und im Vakuum konzentriert. Das Rohprodukt wird an Kieselgel mit Hexan-Ethylacetat-Gemischen chromatographiert. Man erhält 4,91 g Produkt vom Schmelzpunkt 43° C.

d) Butyrat (±)-6

Man löst 4,265 g des kristallisierten Hydroxyesters in 10 ml Pyridin, fügt 4,90 ml Buttersäureanhydrid

zu und rührt 20 Stunden bei 22 ° C. Man versetzt mit Eiswasser, extrahiert mit Dichlormethan und dieses mit Natriumhydrogencarbonat-Lösung, unter Eiskühlung mit 2n-Schwefelsäure und Wasser, trocknet mit Natriumsulfat, konzentriert im Vakuum und chromatographiert an Kieselgel mit Hexan-Ethylacetat-Gemischen. Man erhält 5,03 g Produkt als farbloses Öl.

e) Hemisuccinat (+)-8

Man löst 4,265 g des kristallisierten Hydroxyesters in 10 ml Pyridin, fügt 1,833 g 4-Dimethylaminopyridin und 1,501 g Bernsteinsäurenahydrid dazu und rührt 20 Stunden bei 22 ° C. Dann gibt man in Eiswasser, säuert mit 2n-Schwefelsäure auf pH = 3 an, extrahiert mit Dichlormethan, wäscht mit Natriumchlorid-Lösung, trocknet mit Natriumsulfat und konzentriert im Vakuum. Der Rückstand wird mit Natriumhydrogencarbonat-Lösung aufgenommen, mit Diethylether extrahiert, die Wasserphase auf pH = 3 angesäuert, mit Dichlormethan extrahiert, dieses mit Natriumchlorid-Lösung gewaschen, mit Natriumsulfat getrocknet und im Vakuum konzentriert. Man erhält 5,04 g Produkt als farbloses Öl.

Beispiel A 4

3α-Acxetoxy-7,7-ethylendioxy-cis-bicyclo[3.3.0]octan-2ß-carbonsäuremethylester (+)-2

10,0 g 7,7-Ethylendioxy-3α-hydroxy-cis-bicyclo[3.3.0]octan-2β-carbonsäuremethylester werden unter den Bedingungen des Beispiels A 3 a) umgesetzt und das Rohprodukt an Kieselgel mit Hexan-Ethylacetat-Gemischen chromatographiert. Man erhält 10,28 g Produkt vom Schmelzpunkt 50 ° C.

Beispiel A 5

7,7-(2,2-Dimethyltrimethylendioxy)-3α-hydroxy-cis-bicyclo[3.3.0]octan-2ß-carbonsäure

5,0 des kristallinen Hydroxyesters aus Beispiel A 2 rührt man mit 17,6 ml 1n-Natronlauge 30 Minuten bei 22 ° C, extrahiert mit Ethylacetat, säuert unter Eiskühlung die wäßrige Phase mit 2n-Schwefelsäure auf pH = 3 an, extrahiert mit Dichlormethan, wäscht mit Natriumchlorid-Lösung, trocknet mit Natriumsulfat und konzentriert im Vakuum. Man erhält 4,66 g Produkt, das für die Folgereaktionen genügend rein ist.

Beispiel A 6

3α-Acetoxy-7,7-(2,2-dimethyltrimethylendioxy)-cis-bicyclo[3.3.0]octan-2β-carbonsäure (+)-9

Man löst 3,13 g der Hydroxysäure aus Beispiel A 5 in 15 ml Pyridin und rührt nach Zugabe von 2,74 ml Essigsäureanhydrid 20 Stunden bei 22 ° C. Man gibt Eiswasser dazu, rührt 30 Minuten, extrahiert mit Dichlormethan, wäscht dieses mit Wasser, trocknet mit Natriumsulfat, konzentriert im Vakuum und chromatographiert an Kieselgel mit Hexan-Ethylacetat-Gemischen. Man erhält 3,04 g Produkt als farbloses Öl.

Beispiel A 7

7,7-(2,2-Dimethyl-trimethylendioxy)-3α-hydroxy-cis-bicyclo[3.3.0]octan-2β-carbonsäureethylester

4,78 g der in Beispiel A 5 erhaltenen Hydroxysäure werden mit 50 ml Aceton, 4,90 g Kaliumcarbonat und 5,72 ml Jodethan 2 Tage unter Rückfluß erhitzt. Nach Abkühlen filtriert man, konzentriert das Filtrat im Vakuum, versetzt mit Wasser, extrahiert mit Dichlormethan, trocknet dieses mit Natriumsulfat, konzentriert und reinigt den Rückstand an Kieselgel mit Hexan-Ethylacetat-Gemischen. Man erhält 3,65 g Produkt als farbloses Öl.

Beispiel A 8

3α-Acetoxy-7,7-(2,2-dimethyltrimethylendioxy)-cis-bicyclo[3.3.0]octan-2β-carbonsäureethylester (+)-10

3,10 g des Hydroxyesters aus Beispiel A 7 werden unter den Bedingungen des Beispiels A 3 a) umgesetzt und das Rohprodukt an Kieselgel mit Hexan-Ethylacetat-Gemischen chromatographiert. Man erhält 3,20 g Produkt als farbloses Öl.

Beispiel A 9

7,7-(2,2-Dimethyl-trimethylendioxy)-3α-hydroxy-cis-bicyclo[3.3.0]octan-2ß-carbonsäurebenzylester

3,52 g der im Beispiel A 5 erhaltenen Hydroxysäure werden mit 50 ml Aceton, 3,59 g Kaliumcarbonat und 3,0 ml Benzylchlorid 5 Tage unter Rückfluß erhitzt und wie im Beispiel A 7 beschrieben aufgearbeitet, Man erhält 2,07 g Produkt als farbloses Öl.

Beispiel A 10

3α-Acetoxy-(2,2-dimethyl-trimethylendioxy)-cis-bicyclo[3.3.0]octan-2β-carbonsäurebenzylester (+)-11

1,89 g des Hydroxyesters aus Beispiel A 9 werden unter den Bedingungen des Beispiels A 3 a) umgesetzt und das Rohprodukt an Kieselgel mit Hexan-Ethylacetat-Gemischen chromatographiert. Man erhält 1,78 g Produkt als farbloses Öl.

Beispiel A 11

7,7-(2,2-Dimethyl-trimethylendioxy)-cis-bicyclo[3,3,0]octan-3-on-2-carbonsäurebenzylester

Zu einer Lösung von 2,5 g 7,7-(2,2-Dimethyl-trimethylendioxy)-cis-bicyclo[3.3.0)]octan-3-on-2-carbonsäuremethylester in 50 ml Toluol fügt man 1,2 ml Benzylalkohol und 217 mg Dimethylaminopyridin und erhitzt die Lösung 8 Stunden bei Rückflußtemperatur. Anschließend kühlt man auf 25 °C, fügt gesättigte Natriumchloridlösung zu, extrahiert mit Methylenchlorid, wäscht mit Sole, trocknet mit Magnesiumsulfat und dampft im Vakuum ein. Den Rückstand reinigt man durch Säulenchromatographie an Kieselgel. Mit Hexan/Essigester 8:2 eluiert man 2,6 g der Titelverbindung als farblose Kristalle. Nach Umkristallisation aus Essigester/Hexan erhält man 1,8 g farblose Kristalle vom Schmelzpunkt 78 °C.

Beispiel A 12

7,7-(2,2-Dimethyl-trimethylendioxy)-3α-hydroxy-cis-bicyclo[3.3.0]octan-2β-carbonsäurebenzylester

1,5 g 7,7-(2,2-Dimethyl-trimethylendioxy)-cis-bicyclo[3.3.0]octan-3-on-2-carbonsäurebenzylester (Beispiel 11) werden bei Rückflußtemperatur in 25 ml Methanol gelöst. Anschließend kühlt man auf -40 °C, fügt 470 mg Natriumborhydrid zu und rührt 1 Std. bei -40 °C. Man fügt 3,8 ml Aceton zu, rührt 1 Stunde bei - 40 °C, neutralisiert mit ca. 0,7 ml Eisessig und engt im Vakuum ein. Den Rückstand versetzt man mit 100 ml Wasser, extrahiert mit Methylenchlorid, wäscht mit Sole, trocknet mit Magnesiumsulfat und dampft im Vakuum ein. Nach Chromatographie an Kieselgel erhält mit Hexan/Essigester (6 + 4) 1,4 g der Titelverbindung als farbloses Öl.
IR($CNCl_3$) : 3600, 2960, 2870, 1722, 1447 $cm^{-1}$

Beispiel A 13

7,7-(2,2-Dimethyl-trimethylendioxy)-3α-benzoyloxy-cis-bicyclo[3.3.0]octan-2β-carbonsäurebenzylester

Zu einer Lösung von 1,35 g 7,7-(2,2-Dimethyltrimethylendioxy)3α-hydroxy-cis-bicyclo[3.3.0]octan-2β-carbonsäurebenzylester (Beispiel A 12) in 14 ml Methylenchlorid fügt man bei 0 °C 1.4 ml Pyridin und 0,62 ml Benzoylchlorid und rührt 0,5 Stunden bei 0 °C und 2 Stunden bei 25 °C. Anschließend fügt man 0,2 ml Wasser zu, rührt 1 Stunde, verdünnt mit Methylenchlorid, schüttelt nacheinander mit Wasser, 5%iger Natriumhydrogencarbonatlösung und Wasser. Man trocknet über Magnesiumsulfat und chromatographiert den Eindampfrückstand an Kieselgel. Mit Hexan/Essigester (3 + 2) erhält man 1,36 g der Titelverbindung als farbloses Öl.
IR($CNCl_3$), 2960, 2870, 1725, 1603 $cm^{-1}$

**BEISPIELE**

Die folgenden Ausführungsbeispiele dienen zur Erläuterung des erfindungsgemäßen Verfahrens.

**Beispiel 1**

3 g (±)-3α-Acetoxy-7,7-(2,2-dimethyl-trimethylendioxy)-cis-bicyco[3.3.0]octan-2β-carbonsäure-methylester werden in 100 ml Ethanol gelöst und mit einer Lösung von 1,5 g Lipase-PL aus Acaligenes (Fa. Meita Sangyo) in 1 l 0,1 M Phosphatpuffer pH 7 in einem 2 l - Erlenmeyerkolben vereinigt. Die Suspension wird bei 30 °C auf einem Rotationsschüttler geschüttelt, wobei der Ablauf der Reaktion durch Analyse laufend entnommener Proben verfolgt wird. Nach 21 Stunden Reaktionszeit sind 50% des eingesetzten Substrates umgewandelt. Der Ansatz wird nun 3mal mit Methylisobutylketon extrahiert, die Extrakte vereinigt im Vakuum zur Trockne eingeengt und zur Abtrennung des unumgesetzten Esteracylates über eine Kieselgelsäule chromatographiert (Gradient: Methylenchlorid-Methylenchlorid /10% Aceton). Man erhält 1, 15 g enantiomeren reinen (+)-3α-Hydroxy-7,7-(2,2-dimethyl-trimethylendioxy)-cis-bicyclo[3.3.0]octan-2β-carbon säure-methylester, der nach Kristallisation aus Hexan/Isopropylether bei 64-66 °C schmilzt. ($[\alpha]_D^{20}$ + 26,2 °, c = 1,255 in $HCCl_3$).

**Beispiel 2**

300 mg (±)-3α-Acetoxy-7,7-(2,2-dimethyl-trimethylendioxy)-cis-bicyclo[3.3.0]octan-2β-carbonsäure-methylester werden in 100 ml 0,1 M Phosphatpuffer pH 7 suspendiert, 750 mg Lipase My aus Candida cyclindracea (Fa. Meito Sangyo) zugegeben und die Suspension mit einem Ultra-Turrax homogenisiert. Anschließend wird die Mischung bei Raumtemperatur auf einem Rotationsschüttler geschüttelt. Nach 30 Stunden Reaktionszeit ist das Substrat zu 50% umgesetzt. Der Ansatz wird nun 3mal mit Ether extrahiert, die Extrakte vereinigt und im Vakuum zur Trockne eingedampft. Der hinterbliebene Rückstand wird zur Abtrennung des nicht umgesetzten Ausgangsmaterials mittels eines Lösungsmittelgradienten Methylenchlorid-Methylenchlorid/5% Aceton über eine Kieselgelsäule chromatographiert. Man erhält 105 mg (+) -3α-Hydroxy-7,7-(2,2-dimethyl-trimethylendioxy)-cis-bicyclo[3.3.0]octan-2β-carbonsäure-methylester, der nach Kristallisation aus Hexan einen Schmelzpunkt von 62-63° und einen Drehwert von$[\alpha]_D^{20}$ + 25° (c = 1,01 in $HCCl_3$) aufweist. Durch Vergleichsmessung mit einem authentischen Vergleichsstand wurde hierfür ein Enantiomerenüberschuß von 93,6% festgestellt.

**Beispiel 3**

300 mg (±)-3α-Acetoxy-7,7-(2,2-dimethyl-trimethylendioxy)-cis-bicyclo[3.3.0]octan-2β-carbonsäure-methylester werden in Ethanol gelöst und zu einer Suspension von 750 mg Lipase "Saiken" aus Rhizopus (Fa. Nagase) in 100 ml 0,1 M Phosphatpuffer pH 7 gegeben. Nach 96 Stunden schütteln auf der Schüttelmaschine bei 28 °C wird der Ansatz mit Methylisobutylketon extrahiert, der Extrakt zur Trockne eingeengt und über eine Kieselgelsäule chromatographiert. Man erhält 112 mg (+)-3α-Hydroxy-7,7-(2,2-dimethyl-trimethylendioxy)-cis-bicyclo[3.3.0]octan-2β-carbonsäure-methylester vom Schmelzpunkt 64-65 °C nach Kristallisation aus Hexan. Der Drehwert beträgt $[\alpha]_D^{20}$ +23,8° (c = 1,02 in $HCCl_3$), der Enantiomerenüberschuß nach Vergleichsmessung gegen authentischen Standard 92,5% ee.

**Beispiel 4**

300 mg (±)-3α-Acetoxy-7,7-(2,2-dimethyl-trimethylendioxy)-cis-bicyclo[3.3.0]octan-2β-carbonsäure-methylester werden in 100 ml 0,1 M Phosphatpuffer pH 7 suspendiert, 750 mg α-Chymotrypsin aus Rinderpankreas (Fa. Chemical Dynamics Corporation) zugegeben und mit dem Ultra-Turrax homogenisiert. Anschließend wird die Reaktionsmischung bei 28 °C auf einem Rotationsschüttler geschüttelt, bis das eingesetzte Esteracylat-Substrat zu 50% umgesetzt ist. Danach wird der Ansatz mehrmals mit Methylisobutylketon extrahiert, die vereinigten Extrakte im Vakuum zur Trockne eingeengt und zwecks Abtrennung des unnatürlich konfigurierten, in diesem Falle verseiften Enantiomeren mittels eines Lösungsmittelgradienten Methylenchlorid-Methylenchlorid/5% Aceton über eine Kieselgelsäule chromatographiert. Fraktion 1 enthält das in seiner absoluten Konfiguration dem natürlichen Prostacyclin $PGI_2$ entsprechende, unverseift gebliebene Enantiomere (+)-II. Nach dem Einengen zur Trockne erhält man 115 mg (+)-3α-Acetoxy-7,7-(2,2-dimethyl-trimethylendioxy)-cis-bicyclo[3.3.0]octan-2β-carbonsäure-methylester als nichtkristallisierendes Öl mit einem Drehwert von $[\alpha]_D$ + 2,6° (c = 1,035 in Chloroform).

**Beispiel 5**

Unter den Bedingungen des Beispiels 4 werden 300 mg (±)-3α-Acetoxy-7,7-ethylendioxy-cis-bicyclo-

[3.3.0]octan-2β-carbonsäure-methylester in Phosphatpuffer pH 7 mit 750 mg Substilisin aus Bacillus subtilis (Fa. Boehringer Mannheim) umgesetzt. Nach 10-stündigem Schütteln bei 28 °C wird der Ansatz mit Methylisobutylketon extrahiert, der Extrakt zur Trockne eingeengt und über eine Kieselgelsäule chromatographiert. Mittels des Lösungsmittelgradienten Methylenchlorid-Methylenchlorid/4%Aceton wird als erste Fraktion das unverseifte, dem natürlichen Prostacyclin entsprechend konfigurierte (+)-Enantiomere eluiert. Nach dem Einengen der Fraktion zur Trockne erhält man 130 mg (+)-3α-Acetoxy-7,7-ethylendioxy-cis-bicyclo[3.3.0]octan-2β-carbonsäure-methylester als ölige Flüssigkeit mit einem Drehwert von $[\alpha]_D + 2,4°$ (c = 1,085 in Chloroform).

**Beispiel 6**

Ein 2 l-Erlenmeyerkolben, der 500 ml einer 30 Minuten bei 120 °C im Autoklaven sterilisierten Nährlösung aus 0,1% Pepton, 0,2% Corn steep liquor, 0,5% Glucose und 0,5% Hefeextrakt, pH auf 7.5 eingestellt, enthält, wird mit einer Schrägröhrchenkultur des Stammes Alcaligenes marshallii ATCC 21030 beimpft und 48 Stunden auf einem Rotationsschüttler geschüttelt. Mit 300 ml dieser Anzuchtskultur wird ein 10 l-Fermenter beimpft, der mit 5 l eines sterilisierten Nährmediums der gleichen Zusammensetzung wie die Anzuchtskultur gefüllt ist. Unter Zugabe von Silicon SH als Antischaummittel wird bei 29 °C unter Belüftung (5 l/Min.) und Rühren (220 U/Min.) germiniert. Nach einer Anwachsphase von 36 Stunden wird das Substrat in Form einer steril filtrierten Lösung von 30 g (±)-3α-Acetoxy-7,7-(2,2-dimethyl-trimethylendioxy)-cis-bicyclo[3.3.0]octan-2β-carbonsäure-methylester in 125 ml Ethanol zugegeben und der Reaktionsablauf durch Analyse laufend entnommener Proben verfolgt. Zehn Stunden nach der Substratzugabe sind 50% des eingesetzen Substrates umgesetzt. Der Fermenterinhalt wird nun 4mal mit je 3 l Methylisobutylketon extrahiert, die Extrakte vereinigt und im Vakuum zur Trockne eingedampft. Der verbliebene Rückstand wird in Methanol gelöst und zur Entfernung des Siliconöls durch ein Faltenfilter filtriert. Das Filtrat wird wiederum i.V. zur Trockne gebracht und der Rückstand zur Abtrennung des unumgesetzten unnatürlich konfigurierten (-)-II Enantiomeren (Fraktion 1) über eine Kieselgelsäule chromatographiert (Gradient: Methylenchlorid-Methylenchlorid/20% Aceton). Man erhält in Fraktion 2 nach Kristallisation aus Hexan 10,7 g (+)-3α-Hydroxy-7,7-(2,2-dimethyl-trimethylendioxy)-cis-bicyclo[3.3.0]octan-2β-carbonsäure-methylester vom Schmelzpunkt 63-64 °C, was einer Enantiomerenausbeute von 81,9 % der Theorie entspricht. Der Drehwert beträgt $[\alpha]_D + 26,1°$ (c = 1,3 in Chloroform), der Enantiomerenüberschuß 97,5 % ee.

**Beispiel 7**

Ein 2 l-Erlenmeyerkolben, der 500 ml einer 30 Minuten bei 120 °C im Autoklaven sterilisierten Nährlösung aus 3% Glucose, 1,0% Corn steep liquor, 0,2% $NaNO_3$, 0,1% $KH_2PO_4$, 0,2% $K_2HPO_4$, 0,05% $MgSO_4.7\ H_2O$, 0,002% $FeSo_4.7\ H_2O$ und 0,05% KCl enthält, wird mit einer Schrägröhrchenkultur des Stammes Mucor rouxii (ATCC 8097) beimpft und 2 l/2 Tage bis 30 °C auf einem Rotationsschüttler geschüttelt.
Mit dem Inhalt zweier dieser Anzuchtkulturen wird ein 20 l-Fermenter beimpft, der mit 14 l eines 60 Minuten bei 121 °C und 1,1 bar Überdruck sterilisierten Mediums der gleichen Zusammensetzung wie die Anzuchtkulturen gefüllt ist. Unter Zugabe von Silicon SH also Antischaummittel wird bei 29 °C mit 0,7 bar Überdruck unter Belüftung (15 l/Min.) und Rühren (220U/Min.) germiniert. Nach einer Anwachsphase von 15 Stunden wird das Substrat in Form einer steril filtrierten Lösung von 9 g (±)-3α-Acetoxy-7,7-(2,2-dimethyl-trimethylendioxy)-cis-bicyclo[3.3.0]octan-2β-carbonsäure-methylester in 220 ml Ethanol zugegeben und der Reaktionsablauf durch Analyse laufend entnommener Proben verfolgt.

Zwei Stunden nach der Substratzugabe sind 50% des eingesetzten Substrates umgesetzt. Der Fermenterinhalt wird nun 3mal mit je 10 l Methylisobutylketon extrahiert, die Extrakte vereinigt und im Vakuum zur Trockne eingeengt. Der verbliebene Rückstand wird in Methanol gelöst und zur Entfernung des Siliconöls durch ein Faltenfilter filtriert. Das Filtrat wird wiederum i.V. zur Trockne gebracht und der Rückstand zur Abtrennung des unumgesetzten Ausgansmaterials über eine Kieselgelsäule chromatographiert (Gradient: 5 l Methylenchlorid - 5 l - Methylenchlorid/10% Aceton). Man erhält 3,3 g (+)-3α-Hydroy-7,7-(2,2-dimethyl-trimethylendioxy)-cis-bicyclo[3.3.0]octan-2β-carbonsäure-methylester, der nach Kristallisation aus Hexan/Isopropylether bei 64-65 °C schmilzt. Der Drehwert beträgt $[\alpha]_D^{20}$ +25,7 ° (c = 1,045 in $HCCl_3$). Durch Vergleichsmessung gegen einen authentischen Standard wurde der Enantiomerenüberschuß mit 96% ee bestimmt.

**Beispiel 8**

Ein 2 l - Erlenmeyerkolben, gefüllt mit 500 ml einer sterilen Nährlösung aus 0,1% Pepton, 0,2% Corn steep liquor, 0,5% Glucose und 0,5% Hefeextrakt, pH 7,3, wird mit einer Schrägagarkultur des Stammes Corynebacterium equi (ATCC 21107) beimpft und 48 Stunden bei 30° geschüttelt.
Mit dem Inhalt zweier dieser Anzuchtskulturen wird ein 20 l - Fermenter beimpft, der mit 14 l sterilem Nährmedium der gleichen Zusammensetzung wie die Anzuchtskulturen beschickt ist. Unter Zugabe von Silicon SH als Antischaummittel wird bei 29°C und 0,7 bar Überdruck unter Belüftung (15 l/Min.) und Rühren (220 U/Min.) germiniert. Nach 16 Stunden Anwachszeit wird das Substrat in Form einer steril filtrierten Lösung von 6 g (±)-3α-Acetoxy-7,7-(2,2-dimethyl-trimethylendioxy)-cis-bicyclo[3.3.0]octan-2β-carbonsäure-methylester in 150 ml Ethanol zugegeben und weiter gerührt und belüftet. Drei Stunden nach der Substratzugabe sind 50% des eingesetzten Substrates umgewandelt. Der Ansatz wird nun mit Methylisobutylketon extrahiert und der Extrakt im Vakuum zur Trockne eingeengt. Der verbliebene Rückstand wird durch Methanolbehandlung vom Siliconöl befreit und über eine Kieselgelsäule chromatographiert. Man erhält 1,9 g (+)-3α-Hydroxy-7,7-(2,2-dimethyl-trimethylendioxy)-cis-bicyclo[3.3.0]octan-2β-carbonsäure-methylesterder nach Kristallisation aus Hexan/Isopropylether bei 63-65° schmilzt. Der Drehwert beträgt $[\alpha]_D^{20}$ +25,2° (c=1,04 in $HCCCl_3$).

**Beispiel 9**

Unter den Bedingungen des Beispiels 7 werden mit einer Kultur des Stammes Trichoderma koningi (CBS 85068) 6 g (±)-3α-Acetoxy-7,7-(2,2-dimethyl-trimethylendioxy)-cis-bicyclo[3.3.0]octan-2β-carbonsäure-methylester nach einer Fermentationszeit von 28,5 Stunden zu 2,0 g (+)-3α-Hydroxy-7,7-(2,2-dimethyl-trimethylendioxy)-cis-bicyclo[3.3.0]octan-2β-carbonsäure-methylester umgesetzt. Der Drehwert der aus Hexan kristallisierten Substanz beträgt $[\alpha]_D^{20}$ +24,2° (c=1,015 in $HCCl_3$).

**Beispiel 10**

Unter den Bedingungen des Beispiels 8 werden mit einer Kultur des Stammes Sarcina lutea (ATCC 9341) 6 g (±)-3α-Acetoxy-7,7-(2,2-dimethyl-trimethylendioxy)-cis-bicyclo[3.3.0]octan-2β-carbonsäure-methylester nach 135 Stunden Fermentationszeit zu 1,85 g (+)-3α-Hydroxy-7,7-(2,2-dimethyl-trimethylendioxy)-cis-bicyclo[3.3.0]octan-2β-carbonsäure-methylester umgesetzt. Der Drehwert beträgt $[\alpha]_D^{20}$ +23,6° [c=1,010 in $HCCCl_3$).

**Beispiel 11**

Unter den Bedingungen des Beispiels 7 werden mit einer Kultur des Stammes Penicillium citrinum (ATCC 8506) 6 g 300 mg (±)-3α-Acetoxy-7,7-(2,2-dimethyl-trimethylendioxy)-cis-bicyclo[3.3.0]octan-2β-carbonsäure-methylester in 8 STunden Fermentationszeit zu 2,3 g (+)-3α-Hydroxy-7,7-(2,2-dimethyl-trimethylendioxy)-cis-bicyclo[3.3.0]octan-2β-carbonsäure-methylester umgesetzt. Der Drehwert beträgt $[\alpha]_D^{20}$ +21,8° (c=1,05 in $HCCl_3$).

**Beispiel 12**

Unter den Bedingungen des Beispiels 8 werden mit einer Kultur des Stammes Flavobacterium lutescens (IFO 3085) 6 g (±)-3α-Acetoxy-7,7-(2,2-dimethyl-trimethylendioxy)-cis-bicyclo[3.3.0]octan-2β-carbonsäure-methylester in 47 stunden Fermentationszeit zu 1,8 g (+)-3α-Hydroxy-7,7-(2,2-dimethyl-trimethylendioxy)-cis-bicyclo[3.3.0]octan-2β-carbonsäure-methylester umgesetzt. Der Drehwert beträgt $[\alpha]_D^{20}$ +21.8° (c=1,145 in $HCCCl_3$).

**Beispiel 13**

300 mg (±)-3α-Butyryloxy-7,7-(2,2-dimethyl-trimethylendioxy)cis-bicyclo[3.3.0]octan-2β-carbonsäure-methylester werden in 9 ml Ethanol gelöst und mit einer Lösung von 750 mg Lipase "Saiken" aus Rhizopus (Fa. Nagase) in 100 ml 0,1 M Phosphatpuffer pH 7 vereinigt. Die Suspension wird 150 Stunden bei 28°C auf einem Rotationsschüttler geschüttelt und anschließend mit Methylisobutylketon extrahiert. Der Extrakt wird im Vakuum zur Trockne eingeengt und der Rückstand zu Abtrennung des unumgesetzten Esteracylates über eine Kieselgelsäule chromatographiert. Man erhält 95 mg (+)-3α-Hydroxy-7,7-(2,2-dimethyl-trimethylendioxy)-cis-bicyclo[3.3.0]octan-2β-carbonsäure-methylester mit einem Drehwert von $[\alpha]_D^{20}$ + 22,8° (c=0,905 in $HCCl_3$).

**Beispiel 14**

Unter den Bedingungen des Beispiels 13 werden 300 mg (±)-3α-Butyryloxy-7,7-(2,2-dimethyl-trimethylendioxy)-cis-bicyclo[3.3.0]octan-2β-carbonsäure-methylester in Phosphatpuffer pH 7 in Gegenwart von 750 mg Alcalase T (Fa. Novo Industrias) umgesetzt. Nach 2,5 Stunden schütteln bei 28°C wird der Ansatz mit Methylisobutylketon extrahiert und der Extrakt an Kieselgel chromatographiert. Man erhält 100 mg (-)-3α-Hydroxy-7,7-(2,2-dimethyl-trimethylendioxy)-cis-bicyclo[3.3.0]octan-2β-carbonsäure-methylester mit einem Drehwert von$[\alpha]_D^{20}$ -24,9° c=1,020 in $HCCl_3$).

**Beispiel 15**

300 mg (±)-3α-Dimethylacetoxy-7,7-(2,2-dimethyl-trimethylendioxy)-cis-bicyclo[3.3.0]octan-2β-carbonsäure-methylester werden in 9 ml Ethanol gelöst und mit einer Lösung von 1,5 ml 1:40-verdünnter Esterase aus Schweineleber (Fa. Boehringer) in 100 ml 0,1 M Phosphatpuffer pH 7 vereinigt. Die Mischung wird 1 Stunde bei 28° auf der Schüttelmaschine geschüttelt, anschließend mit Methylisobutylketon extrahiert und der im Vakuum eingedampfte Extrakt über eine Kieselgelsäule chromatographiert. Man erhält 114 mg (+)-3α-Hydroxy-7,7-(2,2-dimethyl-trimethylendioxy)-cis-bicyclo[3.3.0]octan-2β-carbonsäure-methylester als Öl, das langsam durchkristallisiert. Fp.64-65°; $[\alpha]_D^{20}$ +25,1° (c=1,010 in $HCCL_3$).

**Beispiel 16**

300 mg (±)-3α-Carboxypropionyloxy-7,7-(2,2-dimethyl-trimethylendioxy)-cis-bicyclo[3.3.0]octan-2β-carbonsäure-methylester werden in 9 ml Ethanol gelöst und mit einer Lösung von 750 mg Lipase "Sclerotinia" (Fa. Nagase) in 100 ml 0,1 M Phosphatpuffer pH 7 vereinigt. Nach 30 stündigem Schütteln auf der Schüttelmaschine bei 28°C wird der pH-Wert der Lösung mit 0,1 n NaOH auf pH 9,0 eingestellt, anschließend mit Methylisobutylketon extrahiert und der Extrakt im Vakuum zur Trockne eingeengt. Man erhält 105 mg (+)-3α-Hydroxy-7,7-(2,2-dimethyl-trimethylendioxy)-cis-bicyclo[3.3.0]octan-2β-carbonsäure-methylester mit einem Drehwert von $[\alpha]_D^{20}$ +24,0° in $HCCl_3$).

**Beispiel 17**

300 mg (±)-3α-Acetoxy-7,7-(2,2-dimethyl-trimethylendioxy)-cis-bicyclo[3.3.0]octan-2β-carbonsäure-ethylester wurden in 9 ml Ethanol gelöst und mit einer Lösung von 750 mg Lipase aus Schweinepankreas (Fa. Chemical Dynamics Corporation) in 100 ml Phosphatpuffer pH 7 vereinigt. Die Lösung wird bei 28°C auf einem Rotationsschüttler geschüttelt, wobei der Ablauf der Reaktion durch Analyse laufend entnommener Proben verfolgt wird. Nach 1 Stunde Reaktionszeit sind 50% des eingesetzten Substrates umgewandelt. Der Ansatz wird nun 3mal mit Methylisobutylketon extrahiert, die Extrakte vereingt, im Vakuum zur Trockne eingeengt und zur Abtrennung des unumgesetzten Esteracylates über eine Kieselgelsäule chromatographiert (Gradient: Methylenchlorid-Methylenchlorid/10%Aceton). Man erhält 110 mg (+)-3α-Hydroxy-7,7-(2,2-dimethyl-trimethylendioxy)-cis-bicyclo[3.3.0]octan-2β-carbonsäure-ethylester mit einem Drehwert von $[\alpha]_D^{20}$ +23,8° c=1,215 in $HCCl_3$).

**Beispiel 18**

Unter den Bedingungen des Beispiels 17 werden 300 mg (±)-3α-Acetoxy-7,7-(2,2-dimethyl-trimethylendioxy)-cis-bicyclo[3.3.0]octan-2β-carbonsäure-ethylester 1 Stunde lang mit einer Lösung von 1,5 ml (1:40 verdünnt) Esterase aus Schweineleber (Fa.Boehringer Mannheim) in 100 ml Phosphatpuffer pH 7 behandelt. Nach Extraktion mit Methylisobutylketon und Chromatographie über eine Kieselgelsäule erhält man 118 mg (+)-3α-Hydroxy-7,7-(2,2-dimethyl-trimethylendioxy)-cis-bicyclo[3.3.0]octan-2β-carbonsäure-ethylester mit einem Drehwert von $[\alpha]_D^{20}$ +24,3° c=1,075 in $HCCl_3$).

**Beispiel 19**

Unter den Bedingungen des Beispiels 17 werden 300 mg (±)-3α-Acetoxy-7,7-(2,2-dimethyl-trimethylendioxy)-cis-bicyclo[3.3.0]octan-2β-carbonsäure-benzylester 1 Stunde lang mit einer Lösung von 750 mg Lipase-PL aus Alcaligenes (Fa. Meito Sangyo) in 100 ml Phosphatpuffer pH 7 behandelt. Danach wird der Ansatz mit Methylisobutylketon extrahiert, der Extrakt zur Trockne eingeengt und der Rückstand über eine Kieselgelsäule chromatographiert. Man erhält 105 mg (+)-3α-Hydroxy-7,7-(2,2-dimethyl-trimethylendioxy)-

cis-bicyclo[3.3.0]octan-2β-carbonsäure-benzylester mit einem Drehwert von $[\alpha]_D^{20}$ +23,9° (c = 1,045 in $HCCl_3$).

**Beispiel 20**

Unter den Bedingungen des Beispiels 17 werden 300 mg (±)-3α-Acetoxy-7,7-(2,2-dimethyl-trimethylendioxy)-cis-bicyclo[3.3.0]octan-2β-carbonsäure-benzylester 1 Stunde lang mit einer Lösung von 750 mg Lipase My aus Candida cyclindracea (Fa. Meito Sangyo) in 100 ml Phosphatpuffer pH 7 behandelt. Danach wird der Ansatz mit Methylisobutylketon extrahiert, der Extrakt zur Trockne eingeengt und der Rückstand über eine Kieselgelsäule chromatographiert. Man erhält 115 mg (+)-3α-Hydroxy-7,7-(2,2-dimethyl-trimethylendioxy)-cis-bicyclo[3.3.0]octan-2β-carbonsäure-benzylester mit einem Drehwert von $[\alpha]_D^{20}$ +24,5° (c = 1,145 in $HCCl_3$).

**Beispiel 21**

Unter den Bedingungen des Beispiels 8 werden mit einer Kultur des Stammes Alcaligenes paradoxus (ATCC 17713) 6 g (±)-3α-Acetoxy-7,7-(2,2-dimethyl-trimethylendioxy)-cis-bicyclo[3.3.0]octan-2β-carbonsäure -methylester umgesetzt. Vier Stunden nach Zugabe des Substrates sind 50% des eingesetzten Racemats umgewandelt. Der Ansatz wird mit Methylisobutylketon extrahiert und der Extrakt im Vakuum zur Trockne eingeengt. Der verbliebene Rückstand wird zwecks Abtrennung des unnatürlich konfigurierten, verseiften Enantiomeren über eine Kieselgelsäule chromatographiert. Hierbei erscheint in Fraktion 1 das in seiner absoluten Konfiguration dem natürlichen Prostacyclin $PGI_2$ entsprechende, unverseift gebliebene Enantiomere (+)-II. Nachdem man die Fraktion zur Trockne eingeengt hat, erhält man 2,4 g (+)-3α-Acetoxy-7,7-(2,2-dimethyl-trimethylendioxy)-cis-bicyclo[3.3.0]octan-2β-carbonsäure-methylester als nichtkristallisierendes Öl mit einem Drehwert von $[\alpha]_D$ +2,2° (c = 1,11 in Chloroform).

**Patentansprüche**

1. Verfahren zur Herstellung von optisch aktiven (+)-Bicyclo[3.3.0]octanol-derivaten der Formel (+)-I,

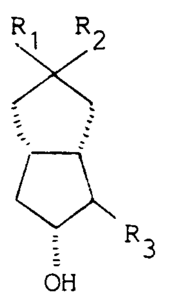

(+)-I,

worin
$R_1$ und $R_2$ gemeinsam ein Sauerstoffatom oder den zweibindigen Rest -O-X-O mit X als gerad- oder verzweigtkettigem Alkylen mit 1-7 C-Atomen oder $R_1$ und $R_2$ jeweils den Rest $OR_5$ mit $R_5$ als gerad- oder verzweigtkettigem Alkyl mit 1-7 C-Atomen und $R_3$ den Rest COOZ mit Z als Wasserstoffatom, gerad- oder verzweigtkettiges Alkyl mit 1-7 C-Atomen, Cycloalkyl mit 3-6 C-Atomen, Phenyl oder Aralkyl mit 7-10 C-Atomen oder
$R_3$ den Rest - $(CH_2)_n$-O-$COR_4$ mit n in der Bedeutung 1-4 und $R_4$ als gerad- oder verzweigtkettigem Alkyl mit 1-7 C-Atomen, Cycloalkyl mit 3-6 C-Atomen, Phenyl oder Aralkyl mit 7-10 C-Atomen, bedeuten,
dadurch gekennzeichnet, daß man racemische 3α-Acyloxy-cis-bicyclo[3.3.0]octan-derivate der Formel (±)-II

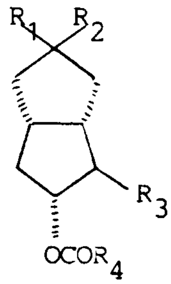

(±)-II,

worin $R_1$, $R_2$, $R_3$ und $R_4$ die oben angegebenen Bedeutungen haben, enzymatisch oder mikrobiologisch einer stereospezifischen Acylat-Hydrolyse unterwirft und das entstehende (+)-Bicyclo-[3.3.0]-octanolderivat (+)-I vom unverseiften Bicyclo[3.3.0]-octanol-acylat der Formel (-)-II abtrennt oder das unverseifte Enantiomere (+)-II vom verseiften Bicyclo[3.3.0]octanolderivat (-)-I abtrennt und anschließend einer chemischen Acylat-Hydrolyse unterwirft.

**2.** Verfahren nach Anspruch 1, dadurch gekennzeichnet, daß man als Enzyme Lipase-PL aus Alcaligenes, Lipase My aus Candida cylindracea, Lipase Saiken aus Rhizopus, Lipase Sclerotinia, α-Chymotrypsin aus Rinderpankreas, Alcalase T, Esterase aus Schweineleber, Lipase aus Schweinepankreas oder Subtilisin aus Bacillus subtilis in gelöster, suspendierter oder an BrCN-aktivierter Sepharose oder an Oxiranacrylperlen immobilisierter Form verwendet.

**3.** Verfahren nach Anspruch 1, dadurch gekennzeichnet, daß man als Mikroorganismen Alcaligenes marshallii, Mucor rouxii, Coryne bacterium equi, Trichoderma koningi, Sarcina lutea, Penicillium citrinum Flavobacterium lutescens oder Alcaligenes paradoxus verwendet.

**4.** Verfahren nach Anspruch 3, dadurch gekennzeichnet, daß man als Enzyme die aus den in Anspruch 3 genannten Mikroorganismen isolierten Enzyme in gelöster, suspendierter oder immobilisierter Form verwendet.

**Claims**

**1.** Process for the manufacture of optically active (+)-bicyclo[3.3.0]octanol derivatives of the formula (+)-I

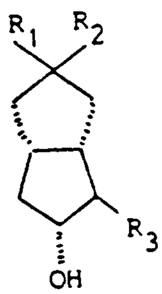

(+)-I,

wherein $R_1$ and $R_2$ together represent an oxygen atom or the bivalent radical -O-X-O- wherein X is straight-chained or branched alkylene having from 1 to 7 carbon atoms, or each of $R_1$ and $R_2$ represents the radical $OR_5$ wherein $R_5$ is straight-chained or branched alkyl having from 1 to 7 carbon atoms, and $R_3$ represents the radical COOZ wherein Z is a hydrogen atom, straight-chained or branched alkyl having from 1 to 7 carbon atoms, cycloalkyl having from 3 to 6 carbon atoms, phenyl or aralkyl having from 7 to 10 carbon atoms, or $R_3$ represents the radical $-(CH_2)_n-O-COR_4$ wherein n is from 1 to 4 and $R_4$ is straight-chained or branched alkyl having from 1 to 7 carbon atoms, cycloalkyl having from 3 to 6 carbon atoms, phenyl or aralkyl having from 7 to 10 carbon atoms, characterised in that racemic 3α-acyloxy-cis-bicyclo[3.3.0]octane derivatives of the formula (+)-II

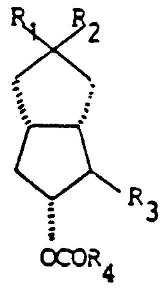

(±)-II,

wherein $R_1$, $R_2$, $R_3$ and $R_4$ have the meanings given above, are subjected enzymatically or microbiologically to stereospecific acylate hydrolysis, and the resulting (+)-bicyclo[3.3.0]octanol derivative (+)-I of the unhydrolysed bicyclo[3.3.0]octanol acylate of the formula (-)-II is separated or the unhydrolysed enantiomer (+)-II of the hydrolysed bicyclo[3.3.0]octanol derivative (-)-I is separated and then subjected to chemical acylate hydrolysis.

**2.** Process according to claim 1, characterised in that there are used as enzymes lipase-PL from Alcaligenes, lipase My from Candida cylindracea, lipase Saiken from Rhizopus, lipase Sclerotinia, α-chymotrypsin from bovine pancreas, alcalase T, esterase from pig's liver, lipase from pig's pancreas or subtilisin from Bacillus subtilis in dissolved or suspended form or in a form immobilised on BrCN-activated Sepharose or on oxiranacryl beads.

**3.** Process according to claim 1, characterised in that there are used as microorganisms Alcaligenes marshallii, Mucor rouxii, Corynebacterium equi, Trichoderma koningi, Sarcina lutea, Penicillium citrinum, Flavobacterium lutescens or Alcaligenes paradoxus.

**4.** Process according to claim 3, characterised in that there are used as enzymes the enzymes isolated from the microorganisms mentioned in claim 3, in dissolved, suspended or immobilised form.

**Revendications**

**1.** Procédé pour préparer des dérivés optiquement actifs de (+) -bicyclo[3.3.0.]octanol de formule (+) - I.

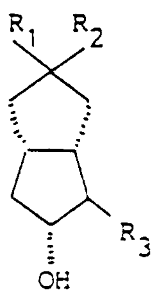

(+)-I,

dans laquelle : $R_1$ et $R_2$ forment ensemble un atome d'oxygène ou représentent le reste dyvalent -O-X-O-, dans lequel X représente un groupe alkylène linéaire ou ramifié ayant 1 à 7 atomes de carbone , ou bien $R_1$ et $R_2$ représentent chacun le reste OR dans lequel $R_5$ représente un groupe alkyle linéaire ou ramifié ayant 1 à 7 atomes de carbone, et $R_3$ représente le reste COOZ, dans lequel Z représente un atome d'hydrogène, un groupe alkyle linéaire ou ramifié ayant 1 à 7 atomes de carbone, cycloalkyle ayant 3 à 6 atomes de carbone, phényle ou aralkyle ayant 7 à 10 atomes de carbone ou

$R_3$ représente le reste $-(CH_2)_n-O-COR_4$ dans lequel n vaut 1 à 4 et $R_4$ représente un groupe alkyle linéaire ou ramifié ayant 1 à 7 atomes de carbone, cycloalkyle ayant 3 à 6 atomes de carbone, phényle ou aralkyle ayant 7 à 10 atomes de carbone, procédé caractérisé en ce qu'on soumet des dérivés racémiques de 3α-acyloxy-cis-bicyclo[3.3.0]octane, de formule (±) -II

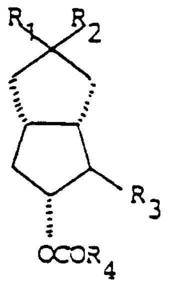

(±)-II,

[dans laquelle $R_1$, $R_2$, $R_3$, et $R_4$ ont les sens précités ] à une hydrolyse stéréospécifique, enzymatique ou microbiologique, de groupe acylate et et l'on sépare le dérivé dans de (+)-bicyclo-[3.3.0]octanol (+)-I-résultant de l'acylate non saponifié de bicyclo [3.3.0]-octanol de formule (-)-II, ou bien l'on sépare l'énantiomère non saponifié (+)-I- du dérivé saponifié de bicyclo[3.3.0] octanol (-)-I et on le soumet ensuite à une hydrolyse chimique de groupe acylate.

**2.** Procédé selon la revendication 1, caractérisé en ce qu'on utilise comme enzyme de la Lipase-PL provenant d'alcaligenes, de la Lipase My de Candida cylindracea, de la Lipase Saiken de Rhizopus, de la Lipase sclérotinia, de l'α-chymotrypsine provenant de pancréas de bovin, de l'alcalase T, de l'estérase de foie de porc, de la Lipase de pancréas de porc ou de la subtilisine de Bacillus subtilis, sous forme dissoute, en suspension ou sous forme immobilisée sur du "Sépharose" activé par BrCN ou sur des perles d'acrylate d'oxyranne.

**3.** Procédé selon la revendication 1 , caractérisé en ce qu'on utilise comme micro-organismes Alcaligenes marshallii, Mucor rouxii, Coryne bacterium equi, Trichoderma koningi, Sarcina lutea, Penicillium citrinum, Flavobacterium lutescens ou Alcaligenes paradoxus.

**4.** Procédé selon la revendication 3, caractérisé en ce que l'on utilise comme enzyme les enzymes, isolées des micro-organismes cités à la revendication 3, et qui sont sous forme dissoute, en suspension ou sous forme immobilisée.